# EUROPEAN PATENT APPLICATION

(11) **EP 1 160 316 A1**
(43) Date of publication of application: **05.12.2001**
(21) Application number: 01113322.0
(22) Date of filing: 31.05.2001
(51) Int. Cl.: C12N 5/06, C12N 5/10, A01K 67/027, C12Q 1/02

(54) **Autonomously growing, invasive fibroblast derived cell line**

(30) Priority: 31.05.2000 EP 00111698
(71) Applicant: BIOTECTID GmbH, 04317 Leipzig (DE)
(72) Inventor: Sack, Ulrich, Dr., 04275 Leipzig (DE); Lehmann, Jörg, Dr., 04457 Mölkau (DE)
(74) Representative: VOSSIUS & PARTNER

(57) **Abstract**

The present invention relates to an autonomously growing cell displaying S-100, murine MHC class I, and secreting murine interleukin-6 comprising co-cultivating connective tissue fibroblasts obtained from a mammal with connective tissue diseases and fibroblasts from mice for at least 6 hours under tissue culture conditions. The cell of the invention is particularly useful in assessing the onset and/or the development of joint destruction, articular destruction and cartilage destruction. It thus forms a basis for a model for studying rheumatoid arthritis. In a preferred embodiment, the cell of the invention is produced by the cell line deposited with DSMZ (Deutsche Sammlung von Mikroorganismen und Zellkulturen, Braunschweig) under accession number DSM ACC 2455 on May 3, 2000.

## Description

The present invention relates to an autonomously growing cell displaying S-100, murine MHC class I, and secreting murine interleukin-6, which is obtainable by co-cultivating synovial membrane fibroblasts obtained from a mammal with rheumatoid arthritis and fibroblasts from mice. The cell of the invention is particularly useful in assessing the onset and/or the development of joint destruction, articular destruction and cartilage destruction. It thus forms a basis for a model for studying rheumatoid arthritis. In a preferred embodiment, the cell of the invention is produced by the cell line deposited with DSMZ (Deutsche Sammlung von Mikroorganismen und Zellkulturen, Braunschweig) under accession number DSM ACC 2455 on May 3, 2000.

Arthritis in humans is a wide-spread disease that is usually progressive with age. Despite considerable efforts undertaken, a reliable cure for arthritis is not in sight. For a better understanding of the nature of the disease, investigators have relied, inter alia, on animal models in the past. Arthritis in rodents induced by immunization with type II collagen (Cll) is a well established model with similarities to the human rheumatoid arthritis (RA). However, like other arthritis models also the Cll-arthritis is not directly comparable to human RA (Klareskog, 1989). Furthermore, most arthritis models require several weeks for investigation, have an incidence of less than 100%, cannot be induced in mice but in rats, and do not allow investigations independent of the immune system.

Of the several cell types contained within the RA synovial membranes (SM), fibroblasts have received increasing attention with regard to their potential arthritogenicity (Müller-Ladner et al., 1996). In SCID mouse transfer protocols, for example, fibroblasts were shown to be invasive to co-implanted cartilage (Müller-Ladner et al., 1997). As SCID mice are unable to reject allografts (Bosma et al. 1983), they are a useful tool for research using human tissues and cells in an in-vivo system. The present inventors, some time ago, decided to transfer cells from RA patients directly into the murine knee joint. This provides an interesting model of destructive action of human RA pannus inside an animal (Sack et al., 1994a).

The arthritis induced by unilateral grafting of synovial membrane explants or synovial fibroblasts of rheumatoid arthritis (RA) patients induces a lymphocyte-independent arthritis in the injected knee joint of the recipient SCID mice (Sack et al., 1994a; Lehmann et al., 2000). Following the initial phase, instigated by the presence of human cells, the inflammatory process is taken over by murine cells (Sack et al., 1996), the latter being most likely (and alone) responsible for the spreading of the original monoarticular arthritis in a systemic oligoarthritic process (Sack et al., 1999). Although human cells remain long detectable within the primary engrafted joint, the destruction areas are mainly enriched with murine cells. Fibroblasts are known to play a central role in arthritic joint destruction (Müller-Ladner et al., 1996) as do macrophages (Sack et al., 1994b; Burmester et al., 1997). This led to the assumption that human cells induce destructive activity in murine macrophages and fibroblasts (Sack et al., 1996; Jorgensen et al., 1996; Jorgensen et al., 1998).

For large scale investigations, the isolation of human cells is, however, unpractical. Furthermore, sample isolation from different patients renders standardization of experimental results more difficult. Accordingly, the technical problem underlying the present invention was to establish means and methods that allow a reproducible and easy assessment of arthritis-related phenomena. The solution to said technical problem is achieved by providing the embodiments characterized in the claims.

Thus, the present invention relates to an autonomously growing cell displaying S-100, murine MHC class I, and secreting murine interleukin-6, which is obtainable by co-cultivating synovial membrane fibroblasts obtained from a mammal with rheumatoid arthritis and fibroblasts isolated from mice for at least 6 weeks under sterile cell culture conditions.

The term "autonomously growing cell" means, in accordance with the present invention, a cell line growing permanently without changing a finally expressed phenotype and without requiring any stimulatory agents except standard culture media.

The surface markers S-100 and murine MHC class I are well known in the art and described, for example, for S-100 in arthritic tissue by (Mohr et al., 1985). MHC class I molecules are common to prove strain and species specificity.

The term "sterile cell culture conditions" is well understood in the art. Briefly, said mammalian cells are cultured in a suitable medium, typically at about 37°C and in an atmosphere of about 5-10 % CO₂, under conditions that avoid contamination by unwanted viable cells such as bacteria or yeast cells.

The cell line of the present invention is obtainable by the referenced method. However, the present invention also includes other methods for establishing the cell of the invention. The cell of the present invention, although autonomously growing, differs from fibroblast tumor cell lines by its non-metastastic invasive growth character.

It is also important to note that synovial membrane fibroblasts may be obtained from more than one mammal and used in the co-cultivation process with murine fibroblasts. The cell of the invention preferably envisages that said fibroblasts are obtained from one mammal and one mouse strain, respectively. However, it is also possible to use fibroblasts from different mouse strains. Exemplary mouse strains that may be used in accordance with the present invention are C.B.17/lcrCrl-scid mice and BALB/c mice.

In accordance with the present invention, it could surprisingly be demonstrated that by interactions between RA synovial fibroblasts, e.g. from humans, and murine fibroblasts, an appropriate cell useful for establishing a model for investigating mechanisms underlying arthritis could be established. Experimentally, an in vitro co-culture system was developed, using RA SM fibroblast lines (derived from the synovial tissues of different RA patients) and mouse fibroblasts derived from SCID or BALB/c mice. Murine fibroblasts (10⁶ per well) were allowed to adhere in 2 ml culture medium (RPMI 1640, 100 U/ml penicillin, 60 U/ml streptomycin, 10 % FCS) in a 6 well culture plate. The cells were then washed with culture medium at 37 °C, and 10⁵ human fibroblasts were added in 2 ml fresh culture medium. The human fibroblasts were set directly onto the murine fibroblasts.
Beginning several weeks after starting co-culture, a murine cell line arose exhibiting an autonomously growing phenotype, expressing antigens S-100, murine MHC class I, and secreting murine interleukin-6.

In accordance with the present invention, it was found that injection of the cells of the invention into knee joints of SCID mice induces a rapid destructive arthritis. Accordingly, the cell of the present invention proves that arthritis can be induced in mice with a very high incident of more than 90% and preferably of 100%. This property is surprising and was not deducible in any way from the published prior art. Of interest for investigators is that joint destruction is extremely fast at in the range of about 2 weeks. The implanted joints show erosion of cartilage and bone. Furthermore, the joint swelling is sustained by the presence of a highly proliferative tissue with focal infiltration of joint, surrounding connective tissue, and muscles. Since this result is reproducible, it allows an easy assessment by visual means. It has also been found in accordance with the present invention that, upon administration of the cell of the invention, murine macrophages can be detected close to the degrated cartilage and bone. In view of the above, the cell of the invention allows the establishment of a model for studying in more depth the mechanisms leading to the onset and/or development of arthritis which allows to investigate destructive processes independent of the presence of immune cells.

Another advantage of the cell of the invention over cells isolated from arthritis patients is that the cells can be stably maintained for years. Alternatively, the cells can be frozen and thus stored for a long time. When needed, the cells can be thawed according to standard protocols.

Preferably, the cell of the invention has an aneuploid genotype.

In a further preferred embodiment in accordance with the present invention said mammal from which the synovial membrane fibroblasts are obtained is a human. It is understood that the human is required to give informed consent prior to the withdrawal of the cells.

In a further preferred embodiment of the present invention, the co-cultivation of human synovial and murine fibroblasts is effected for at least 12 weeks.
The 12 weeks period is preferred, since this time period could be shown in all the successful experiments carried out so far to give rise to the autonomously growing cell of the invention.

It is also preferred in accordance with the invention that said co-cultivation is effected in a 6 well microtiter plate.
The 6 well microtiter plate is particularly handy for the generation of the cell of the invention. Alternatively, other adequate cell culture equipment could be used.

In another preferred embodiment of the cell of the invention, about 10⁶ murine fibroblasts and about 10⁶ human synovial fibroblasts are seeded into one well of said 6 well microtiter plate at the outset of said co-cultivation.

In a different preferred embodiment, the cell of the invention is produced by the cell line deposited with DSMZ (Deutsche Sammlung von Mikroorganismen und Zellkulturen, Braunschweig) under accession number DSM ACC 2455 on May 3, 2000. The deposit was made under the requirements of the Budapest Treaty. The deposit has been made by Universität Leipzig, Institut für Klinische Immunologie und Transfusionsmedizin, Johannisallee 30, 04103 Leipzig. Universität Leipzig authorizes the applicant to refer to the material deposited with DMSZ-Deutsche Sammlung von Mikroorganismen und Zellkulturen GmbH on May 3, 2000 under accession number DSM ACC2455 in the European patent application "Autonomously growing, invasive fibroblast derived cell line" and gives its unreserved and irrevocable consent to said material being made available to the public in accordance with Rule 28(1) d) EPC and, if admissible, Rule 28(4) EPC.

The present invention relates in a further preferred embodiment to a cell that comprises externally introduced genetic material.

The externally introduced genetic material is preferably DNA, such as cDNA, encoding a (poly)peptide, wherein said DNA is comprised in a vector.

The (poly)peptide is advantageously a cytokine, a regulator of apoptosis, a receptor antagonist, a bioactive peptide, a growth regulatory molecule or a molecule involved in the regulation of differentiation.

The vectors may particularly be plasmids, cosmids, viruses or bacteriophages used conventionally in genetic engineering. Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the genetic material into targeted cell population. Methods which are well known to those skilled in the art can be used to construct recombinant viral vectors; see, for example, the techniques described in Sambrook et al., Molecular Cloning A Laboratory Manual, Cold Spring Harbor Laboratory (1989) N.Y. and Ausubel et al., Current Protocols in Molecular Biology, Green Publishing Associates and Wiley Interscience, N.Y. (1989). Alternatively, the genetic material and vectors can be reconstituted into liposomes for delivery to target cells. The vectors containing the genetic material can be transferred into the host cell by well-known methods, which vary depending on the type of cellular host. For example, calcium phosphate or DEAE-Dextran mediated transfection or electroporation may be used for eukaryotic cellular hosts; see Sambrook, supra.
Such vectors may comprise further genes such as marker genes which allow for the selection of said vector in a suitable host cell and under suitable conditions. Preferably, the genetic material is operatively linked to expression control sequences allowing expression in eukaryotic cells. Expression of said genetic material comprises transcription of the genetic material into a translatable mRNA. Regulatory elements ensuring expression in eukaryotic cells, preferably mammalian cells, are well known to those skilled in the art. They usually comprise regulatory sequences ensuring initiation of transcription and, optionally, a poly-A signal ensuring termination of transcription and stabilization of the transcript, and/or an intron further enhancing expression of said polynucleotide. Additional regulatory elements may include transcriptional as well as translational enhancers, and/or naturally-associated or heterologous promoter regions. Examples for regulatory elements permitting expression in eukaryotic host cells are the AOX1 or GAL1 promoter in yeast or the CMV-, SV40- , RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the polynucleotide of the invention and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including a C- or N-terminal identification peptide imparting desired characteristics, e.g., stabilization or simplified purification of expressed recombinant product. In this context, suitable expression vectors are known in the art such as Okayama-Berg cDNA expression vector pcDV1 (Pharmacia), pCDM8, pRc/CMV, PcDNA1, pcDNA3, the Echo™ Cloning System (Invitrogen), pSPORT1 (GIBCO BRL) or pRevTet-On/pRevTet-Off or pCl (Promega).
Preferably, the expression control sequences will be eukaryotic promoter systems in vectors capable of transforming or transfecting eukaryotic host cells.
As mentioned above, the vector may also be a gene transfer or targeting vector. Gene therapy, which is based on introducing therapeutic genes into cells by ex-vivo or in-vivo techniques is one of the most important applications of gene transfer. Suitable vectors and methods for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Isner, Lancet 348 (1996), 370-374; Muhlhauser, Circ. Res. 77 (1995), 1077-1086; Wang, Nature Medicine 2 (1996), 714-716; WO94/29469; WO 97/00957 or Schaper, Current Opinion in Biotechnology 7 (1996), 635-640, and references cited therein. The polynucleotides and vectors may be designed for direct introduction or for introduction via liposomes, or viral vectors (e.g. adenoviral, retroviral) into the cell.

This preferred embodiment of the present invention may be used to modify the cause of disease processes after administering of said cell into a non-human mammal. In cell culture experiments, interactions with lymphocytes, macrophages, other cells derived from connective tissues, tumor cells such as tumor cells with intercellular matrices and matrix portions can be tested. Further, this embodiment of the invention can be used to test for reactions towards soluble factors such as cytokines, regulators of apoptosis, receptor antagonists, bioactive peptides, growth and differentiation regulators. Furthermore, the cell of the invention can be used to induce production of cytokines such as interleukin-12.

The invention also relates to a cell line comprising the cell of the invention. The term "cell line", with regard to the present invention, is intended to mean cells with a stable genotype and phenotype after finishing at least the induction steps. Preferably, the cell line is the cell line deposited with DSMZ under accession number DSM ACC 2455 on May 3, 2000.

In addition, the invention also relates to a method of studying the onset and/or the development of joint destruction, articular destruction or cartilage destruction in a non-human mammal comprising administering the cell of the invention or the cell line of the invention to said mammal and assessing the onset and/or development of an arthritis.
As has been demonstrated in accordance with the present invention, administration of the cell or cell line of the invention into knee joints of mice leads to joint swelling. By following up the processes following the administration of the cell/cell line and leading to the swelling, for example by histological analyses, the onset and/or development of the arthritis disease state may be unraveled. By elucidating molecular details leading to said onset and/or development, appropriate pharmaceutical agents countering said process may be developed or selected.

If the cell of the invention that is administered is the cell carrying externally introduced genetic material, then the cell line can be used to study a cure for the above-referenced disease states. In particular, if the externally introduced genetic material is under the control of an inducible promoter and the (poly)peptide encoded by the DNA comprised in the vector that was introduced, then after the onset of said disease states, the promoter may be induced. If the (poly)peptide produced on induction of the promoter indeed produces a pharmaceutically useful compound, then the progress of the disease state might be halted or reversed.

Furthermore, the invention relates to a method of generating arthritis in a non-human mammal comprising administering the cell of the invention or the cell line of the invention to said mammal. Preferably, about 10⁵-10⁶ cells are injected preferably into the joint of, preferably, a SCID mouse.

In addition, the invention relates to a method of generating a non-metastizing invasive fibroblastoid tumor in a non-human mammal comprising administering the cell of the invention or the cell line of the invention to said mammal. Preferably, about 10⁵-10⁶ cells are injected; preferably into the joints or subcutaneously. Preferred experimental animals are SCID mice.

This embodiment of the present invention is also designed to eventually develop a cure for arthritis. Due to the fact that the animals injected with the cell of the invention rapidly and reliably develop symptoms of arthritis, highly standardized tests for assessing the development of said symptoms and for developing counteragents thereto may now be advanced.

Additionally, the invention relates to a method of screening for a compound suitable for the prevention or treatment of joint destruction, articular destruction or cartilage destruction comprising (aa) administering the cell of the invention comprising externally introduced genetic material to a non-human mammal wherein said externally introduced genetic material encodes the compound of interest; or (ab) administering the cell of the invention and the compound of interest to a non-human mammal; and (b) assessing whether the compound of interest prevents, reduces or retards joint destruction, articular destruction or cartilage destruction.
In accordance with the method of the invention, one or more compounds may be administered to said non-human mammal. These compounds may either be administered by way of expression within the cell of the invention (alternative (aa)). Alternatively, they may be recombinantly, (semi)synthetically or synthetically produced and administered together with the cell of the invention or before or after the administration of the cell of the invention.

If option (ab) is employed and if the cell of the invention comprises externally introduced genetic material, then said material may or may not encode the compound of interest. If said genetic material encodes a compound of interest, then in option (ab) said compound will be tested together with another compound of interest that is not expressed by the cell of the invention. Thus, a compound synthesized by the cell of the invention as well as the further compound that is also administered to said animal may be assessed for synergistic activities. Of course, if various compounds are either encoded by the externally introduced genetic material or are separately administered to said animals and not encoded by said genetic material comprised in the cell of the invention, such synergies may also be tested.

When assessing the effect of the compound on the onset or development of joint destruction, articular destruction or cartilage destruction, the effects may be compared to those observed in a mammal to which only the cell comprising no externally introduced genetic material and no additional compound was administered. The assessment may, for example, be effected by imaging, histological examination of destruction or measurement of joint swelling.

In a preferred embodiment of the method of the present invention said compound of interest is a proteinase inhibitor, immune modulatory molecule or a regulator of apoptosis, of cell adhesion or of signal transduction. In accordance with the method of the present invention, it is envisaged that in particular anti-inflammatory proteinase inhibitors reduce or prevent joint destruction, articular destruction or cartilage destruction.

In a particularly preferred embodiment of the method of the present invention said immune modulatory molecule is an interleukin.
Moreover, the invention relates to a method of modifying the compound preventing, reducing or retarding joint destruction, articular destruction or cartilage destruction in the method described above as a lead compound to achieve (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi)modified pharmakinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carbon acids, or (iii) esterification of hydroxyl groups to, e.g. phosphates, pyrophosphates or sulfates or hemi succinates, or (iv) formation of pharmaceutically acceptable salts, or (v) formation of pharmaceutically acceptable complexes, or (vi) synthesis of pharmacologically active polymers, or (vii) introduction of hydrophylic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiff's bases, oximes, acetales, ketales, enolesters, oxazolidines, thiozolidines or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

In a preferred embodiment of the method of the invention said modification is effected by peptidomimetics. Methods of refining compounds by peptidomimetics are well known in the art and described, for example, in Ostresh, Methods in Enzymology 267 (1996), 220-234 and Dorner, Bioorg. Med. Chem. 4 (1996), 709-715.

In a preferred embodiment of the method of the invention said compound is a small molecule or a peptide.
The invention furthermore relates to a method of producing a pharmaceutical composition comprising the steps of the method as described herein above and further the steps of formulating the compound of interest with a pharmaceutically acceptable carrier or diluent.

Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. A typical dose can be, for example, in the range of 0.001 to 1000 *µ*g (or of nucleic acid for expression or for inhibition of expression in this range); however, doses below or above this exemplary range are envisioned, especially considering the aforementioned factors. Generally, the regimen as a regular administration of the pharmaceutical composition should be in the range of 1 *µ*g to 10 mg units per day. If the regimen is a continuous infusion, it should also be in the range of 1 *µ*g to 10 mg units per kilogram of body weight per minute, respectively. Progress can be monitored by periodic assessment. Dosages will vary but a preferred dosage for intravenous administration of DNA is from approximately 10⁶ to 10¹² copies of the DNA molecule. The compositions of the invention may be administered locally or systemically. Administration will generally be parenterally, e.g., intravenously; DNA may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents such as interleukins or interferons depending on the intended use of the pharmaceutical composition.

In a preferred embodiment of the method of the invention, 10⁴ to 10⁷ cells are administered to said non-human mammal.

In a particularly preferred embodiment of the method of the invention, 10⁵ to 10⁶ cells are administered to said non-human mammal.

In another preferred embodiment of the method of the invention, said non-human mammal is immunocompromised.

The term "immunocompromised" is well understood in the art. Briefly, due to different spontaneously occurring or intentionally induced alterations, no complete functional immune system is present in these animals. This allows to investigate engrafted autonomously growing or allogeneic cells in these animals.

In a preferred embodiment of the method of the invention, said non-human mammal is a mouse.
In a particular preferred embodiment of the invention, said mouse is a SCID mouse.

In another preferred embodiment of the method of the invention, said non-human mammal is not immunocompromised. In this preferred embodiment, the cell can be tested in animals that are syngeneic with regard to the cell or cell line of the invention. For example, in mice that display the same MHC markers as the cell or cell line of the invention, tumors arising from an engrafting or injection of the cell or cell line of the invention will grow since these mice would not be expected to reject said cells. Experiments in not immunocompromised mammals are also advantageous since they are less expensive than immunocompromised animals. In addition, the facilities to keep these animals need to be less sophisticated.

Furthermore, the invention relates to a method for producing an autonomously growing cell displaying S-100, murine MHC class I, and secreting murine interleukin-6 comprising co-cultivating connective tissue fibroblasts obtained from a mammal with connective tissue diseases and fibroblasts from mice for at least 6 hours under sterile tissue culture conditions.
The method of the invention can be carried out along the teachings provided by the present specification and in particular the examples. It may be expected that in 40 % of all experiments that are carried out on the basis of said teachings and thus without undue burden, the desired cells can be isolated. Advantageously, said co-cultivation is effected in a 6 well microtiter plate.

In a preferred embodiment of the method of the invention, said connective tissue disease is an arthritis.

In another preferred embodiment of the method of the invention, said cell has an aneuploid genotype.

In a further preferred embodiment of the method of the invention, said one or more mammals are humans.

In a preferred embodiment of the method of the invention, the co-cultivation is effected for at least 12 hours.

In another preferred embodiment of the method of the invention, said co-cultivation is effected in RPMI 1640 medium comprising 100 U/ml penicillin, 60 U/ml streptomycin and 10 % fetal calf serum.

In a further preferred embodiment of the method of the invention, at the outset of said co-cultivation, about 10⁶ murine fibroblasts and about 10⁶ human fibroblasts are seeded into the cultivation container.

In another preferred embodiment of the method of the invention, said cell is part of the cell line.

A different embodiment of the present invention relates to a cell produced by the method of the invention.

Finally, the invention relates to a kit comprising the cell or cell line of the invention in one ore more containers.

The containers are advantageously vials, preferably plastic vials. If the cells are to be shipped in a frozen state, cryovials are preferred.

### The figures show:

- Figure 1:: Joint swelling following injection of generated cell line into the murine knee joint, in three different experiments with SCID mice (5 animals per group). Each symbol stands for one experimental group without additional treatment; mean rSD.
- Figure 2:: Joint destruction of a murine (SCID) knee joint 14 days following engrafting invented cell line. Cells invade into articular cartilage (left) and destroy structures of the patella (right).
- Figure 3:: Joint swelling (5 animals per group) following injection of generated cell line into the murine knee joint, reduced by higher but not low concentrations of an antirheumatic drug (asterices indicate p < 0.05).
Each symbol stands for one experimental group without additional treatment; mean tSD.

The examples illustrate the invention.

### Example 1: Generation of human RA synovial fibroblasts

Synovial tissue was obtained by joint surgery from patients with RA who all gave their informed consent and met the American College of Rheumatology 1987 revised criteria for the classification of RA (Arnett et al., 1988). After surgical excision, the samples were stored immediately in sterile isotonic phosphate buffered saline (PBS) containing 100 U/ml penicillin, 60 U/ml streptomycin, and 2.5 *µ*g/ml amphotericin B (all Gibco Life Technologies, Karlsruhe, Germany). The synovial tissue was examined for macroscopic signs of inflammation, i.e. oedema, hyperaemia, and fibrin deposits. Regions showing a high degree of local inflammation were cut into small pieces which were set into tissue culture flasks and allowed to adhere for 30 min. The culture medium (30% RPMI 1640, 30% DMEM [both Gibco], 30% FGM [Clonetics, Verviers, Belgium], 10% fetal calf serum [FCS, Sigma, Deisenhofen, Germany], 100 U/ml penicillin, 60 U/ml streptomycin, 2.5 *µ*g/ml amphothericin B [all Gibco]) was then added, to allow the cells to grow out from the tissue pieces. The tissue was cultured at 37°C, 5% CO₂, and 98% relative air humidity. Contaminating lymphocytes and macrophages were removed by repeated cell culture passaging, with cells being passaged 8 to 10 times. After three passages, the cultures only consisted of fibroblast-like cells, as controlled by morphology and flow cytometry.

### Example 2: Generation of murine fibroblasts

Murine fibroblast-like cells were obtained by outgrowing from small connective tissue pieces derived from the peritoneum or the knee joint of SCID mice or BALB/c mice. In detail, the mouse was sacrificed by cervical dislocation and disinfected in a plunge bath of 70 % ethanol. Thereafter the abdominal skin of the animal was opened using sterile small scissors and the peritoneum was bared aseptically. Than, small pieces of peritoneum-associated connective tissue were excised and transferred under sterile conditions into a tissue culture flask. Alternatively, the knee joint was bared using sterile small scissors and small pieces of joint-associated connective tissue were excised and transferred under sterile conditions into a tissue culture flask. The connective tissue pieces were allowed to adhere for 30 min. The culture medium (30% RPMI 1640, 30% DMEM [both Gibco], 30% FGM [Clonetics, Verviers, Belgium], 10% fetal calf serum [FCS, Sigma, Deisenhofen, Germany], 100 U/ml penicillin, 60 U/ml streptomycin, 2.5 *µ*g/ml amphothericin B [all Gibco]) was then added, to allow the cells to grow out from the tissue pieces. The tissue was cultured at 37°C, 5% CO₂, and 98% relative air humidity. Within 4 to 6 weeks the outgrowing cells formed a monolayer and showed only fibroblast-like morphology.

### Example 3: Co-culture of murine and rheumatoid human fibroblasts

Murine Fibroblasts (10⁶ per well) were allowed to adhere in 2 ml culture medium (RPMI 1640, 100 U/ml penicillin, 60 U/ml streptomycin [all Gibco], 10 % FCS [Sigma]) in a 6-well culture plate (Costar, Bodenheim, Germany). The cells were then washed with culture medium at 37 °C, and 10⁶ human synovial fibroblasts were added in 2 ml fresh culture medium. The human rheumatoid fibroblasts were set directly onto the murine fibroblasts. Culture medium was refreshed weekly.

### Example 4: Arthritis induction

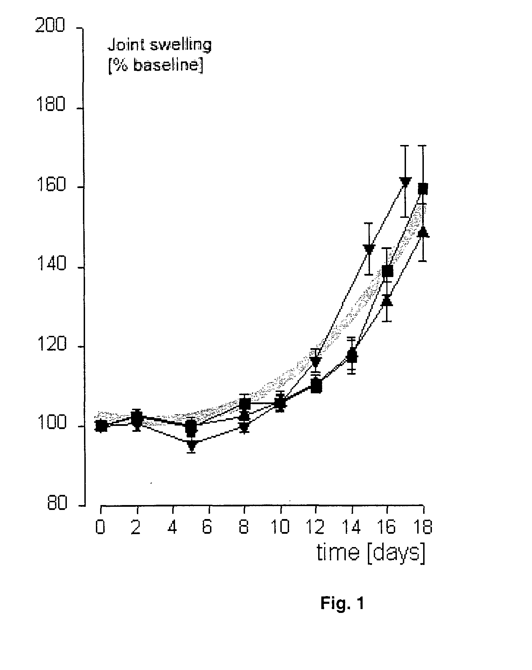

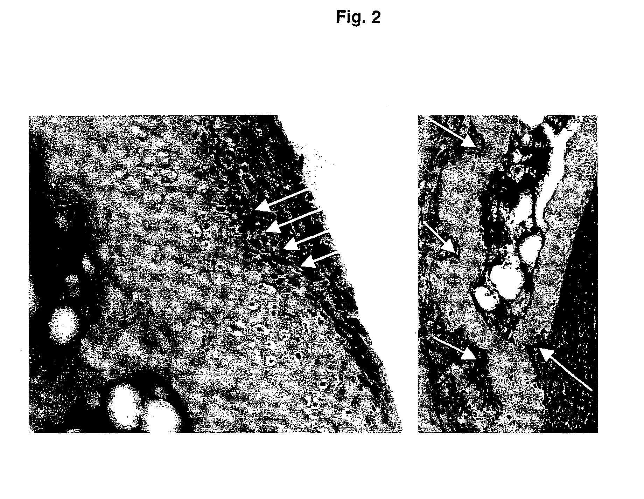

Arthritis was induced by intra-articular injection into SCID mice. Cells (2 x 10⁶ / 20 *µ*l PBS) were injected into the mouse knee joint from an anterolateral position. Joint swelling (both in the iniected and noniniected side) was monitored every week by measuring the diameter with a caliper, until the mice were sacrificed at 2 weeks post-injection (Figure 1). The joints were frozen for preparation of serial cryostat sections, or fixed in 4% paraformaldehyde (Sigma) for preparation of serial paraffin sections, respectively. The extent of joint destruction was assessed histologically in Giemsa stained cryostat sections, as well as in hematoxylin/eosin-stained paraffin sections, according to criteria published before (Sack et al., 1996). A rapid progressive destruction of injected but not of other murine joints was found (Figure 2).

### Example 5: Modulation of arthritis

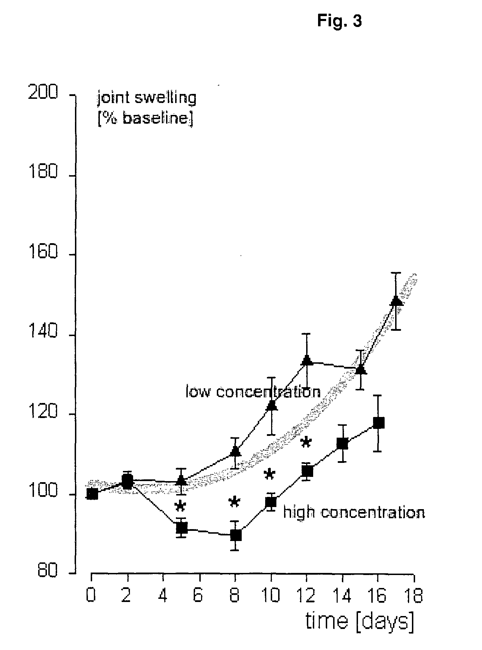

Arthritis induced as shown in example 4 was modified by daily application of an anti-inflammatory remedy. Joint swelling as well as destruction were reduced (Figure 3).

### Reference List

- Anderson. Science 256 (1992): 808-813.
- Arnett FC, Edworthy SM, Bloch DA, McShane DJ, Fries JF, Cooper NS, Healey LA, Kaplan SR, Liang MH, and Luthra HS (1988). The American Rheumatism Association 1987 revised criteria for the classification of rheumatoid arthritis. Arthritis Rheum 31:315-324.
- Austyn JM and Gordon S (1981). F4/80, a monoclonal antibody directed specifically against the mouse macrophage. Eur J Immunol 11:805-815.
- Ausubel et al. Current Protocols in Molecular Biology. Green Publishing Associates and Wiley Interscience, N.Y. (1989).
- Bosma GC, Custer RP, and Bosma MJ (1983). A severe combined immunodeficiency mutation in the mouse. Nature 301:527-530.
- Burmester GR, Stuhlmüller B, Keyszer G, Kinne RW (1997). Mononuclear phagocytes and rheumatoid synovitis. Mastermind or workhorse in arthritis? Arthritis Rheum 40:5-18.
- Dorner, Bioorg. Med. Chem. 4 (1996), 709-715.
- Giordano. Nature Medicine 2 (1996):534-539.
- Hume DA, Robinson AP, MacPherson GG, and Gordon S (1983). The mononuclear phagocyte system of the mouse defined by immunohistochemical localization of antigen F4/80. Relationship between macrophages, Langerhans cells, reticular cells, and dendritic cells in lymphoid and hematopoietic organs. J Exp Med 158:1522-1536.
- Isner. Lancet 348 (1996): 370-374.
- Jörgensen C and Gay S (1998). Gene therapy in osteoarticular diseases: where are we? Immunol Today 19:387-391.
- Klareskog L (1989). What can we learn about rheumatoid arthritis from animal models? Springer Semin Immunopathol 11:315-333.
- Lehmann J, Jungel A, Lehmann I, Busse F, Biskop M, Saalbach A, Emmrich F, and Sack U. Induction of chronic arthritis in knee joint of SCID mice by injection of human synovial membrane-derived fibroblasts from Rheumatoid Arthritis (RA) patients - a novel *in vivo* model for studying the arthritogenic role of RA fibroblasts. submitted
- Mohr W, Kuhn C, Pelster B, and Wessinghage D (1985). S-100 protein in normal, osteoarthrotic, and arthritic cartilage. Rheumatol Int 5:273-277.
- Muhlhauser. Circ. Res. 77 (1995):1077-1086.
- Müller Ladner U, Gay RE, and Gay S (1997). Cellular pathways of joint destruction. Curr Opin Rheumatol 9.213-220.
- Müller Ladner U, Kriegsmann J, Franklin BN, Matsumoto S, Geiler T, Gay RE, and Gay S (1996). Synovial fibroblasts of patients with rheumatoid arthritis attach to and invade normal human cartilage when engrafted into SCID mice. Am J Pathol 149:1607-1615.
- Ostresh, Methods in Enzymology 267 (1996), 220-234
- Sack U, Kuhn H, Ermann J, Kinne RW, Vogt S, Jungmichel D, and Emmrich F (1994a). Synovial tissue implants from patients with rheumatoid arthritis cause cartilage destruction in knee joints of SCID.bg mice. J Rheumatol 21:10-16.
- Sack U, Stiehl P, and Geiler G (1 994b). Distribution of macrophages in rheumatoid synovial membrane and its association with basic activity. Rheumatol Int 13:181-186.
- Sack U, Kuhn H, Kampfer I, Genest M, Arnold S, Pfeiffer G, and Emmrich F (1996). Orthotopic implantation of inflamed synovial tissue from RA patients induces a characteristic arthritis in immunodeficient (SCID) mice. J Autoimmun 9:51-58.
- Sack U, Gunther A, Pfeiffer R, Genest M, Kinne J, Biskop M, Kämpfer I, Krenn V, Emmrich F, and Lehmann J (1999). Systemic Characteristics of Chronic Arthritis Induced by Transfer of Human Rheumatoid Synovial Membrane into SCID Mice (Human/Murine SCID Arthritis). J Autoimmun 13:335-346.
- Sambrook et al. Molecular Cloning A Laboratory Manual. Cold Spring Harbor Laboratory (1989) N.Y.
- Schaper. Circ. Res. 79 (1996):911-919.
- Schaper. Current Opinion in Biotechnology 7 (1996), 635-640.
- Wang. Nature Medicine 2 (1996):714-716.
- WO 94/29469
- WO 97/00957

## Claims

1. An autonomously growing cell displaying S-100, murine MHC class I, and secreting murine interleukin-6 which is obtainable by co-cultivating synovial membrane fibroblasts obtained from a mammal with rheumatoid arthritis and isolated fibroblasts from mice for at least 6 weeks under sterile cell culture conditions.

2. The cell according to claim 1 which has an aneuploid genotype.

3. The cell according to claim 1 or 2, wherein said mammal is a human.

4. The cell according to any one of claims 1 to 3, wherein the co-cultivation is effected for at least 12 weeks.

5. The cell according to any one of claims 1 to 4, wherein said co-cultivation is effected in a 6 well microtiter plate.

6. The cell according to any one of claims 1 to 5, wherein, at the outset of said co-cultivation, about 10⁶ murine fibroblasts and about 10⁶ mammal fibroblasts are seeded into one well of said 6 well microtiter plate.

7. The cell according to any one of claims 1 to 6 which is produced by the cell line deposited with DSMZ under accession number DSM ACC 2455 on May 3, 2000.

8. The cell according to any one of claims 1 to 7 comprising externally introduced genetic material.

9. A cell line comprising the cell of any one of claims 1 to 8.

10. A method of studying the onset and/or the development of joint destruction, articular destruction or cartilage destruction in a non-human mammal comprising administering the cell of any one of claims 1 to 8 or the cell line of claim 9 to said mammal and assessing the onset and/or development of an arthritis.

11. A method of generating an arthritis in a non-human mammal comprising administering the cell of any one of claims 1 to 8 or the cell line of claim 9 to said mammal.

12. A method of generating a non-metastizing invasive fibroblastoid tumor in a non-human mammal comprising administering the cell of any one of claims 1 to 8 or the cell line of claim 9 to said mammal.

13. A method of screening for a compound suitable for the prevention or treatment of joint destruction, artucular destruction or cartilage destruction comprising
(aa) administering the cell of claim 8 to a non-human mammal wherein said externally introduced genetic material encodes the compound of interest; or
(ab) administering the cell of any one of claims 1 to 8 and the compound of interest to a non-human mammal; and
(b) assessing whether the compound of interest prevent, reduces or retards joint destruction, articular destruction or cartilage destruction.

14. The method of claim 13, wherein said compound of interest is a proteinase inhibitor, immune modulatory molecule, or a regulator of apoptosis, of cell adhesion or of signal transduction.

15. The method of claim 14, wherein said immune modulatory molecule is an interleukin.

16. A method of modifying the compound preventing, reducing or retarding joint destruction, articular destruction or cartilage destruction in the method of claim 13 as a lead compound to achieve
(i) modified site of action, spectrum of activity, organ specificity, and/or
(ii) improved potency, and/or
(iii) decreased toxicity (improved therapeutic index), and/or
(iv) decreased side effects, and/or
(v) modified onset of therapeutic action, duration of effect, and/or
(vi) modified pharmakinetic parameters (resorption, distribution, metabolism and excretion), and/or
(vii) modified physico-chemical parameters (solubility, hygroscopicity, color, taste, odor, stability, state), and/or
(viii) improved general specificity, organ/tissue specificity, and/or
(ix) optimized application form and route
by
(i) esterification of carboxyl groups, or
(ii) esterification of hydroxyl groups with carbon acids, or
(iii) esterification of hydroxyl groups to, e.g. phosphates, pyrophosphates or sulfates or hemi succinates, or
(iv) formation of pharmaceutically acceptable salts, or
(v) formation of pharmaceutically acceptable complexes, or
(vi) synthesis of pharmacologically active polymers, or
(vii) introduction of hydrophylic moieties, or
(viii) introduction/exchange of substituents on aromates or side chains, change of substituent pattern, or
(ix) modification by introduction of isosteric or bioisosteric moieties, or
(x) synthesis of homologous compounds, or
(xi) introduction of branched side chains, or
(xii) conversion of alkyl substituents to cyclic analogues, or
(xiii) derivatisation of hydroxyl group to ketales, acetales, or
(xiv) N-acetylation to amides, phenylcarbamates, or
(xv) synthesis of Mannich bases, imines, or
(xvi) transformation of ketones or aldehydes to Schiff's bases, oximes, acetales, ketales, enolesters, oxazolidines, thiozolidines
or combinations thereof.

17. The method of claim 16, wherein said modification is effected by peptidomimetics.

18. The method of any one of claims 13 to 17, wherein said compound is a small molecule or a peptide.

19. A method of producing a pharmaceutical composition comprising the steps of the method of any one of claims 13 to 18 and further the steps of formulating the compound of interest with a pharmaceutically acceptable carrier or diluent.

20. The method according to any one of claims 10 to 15, wherein 10⁴ to 10⁷ cells are administered to said non-human mammal.

21. The method according to claim 20, wherein 10⁵ to 10⁶ cells are administered to said non-human mammal.

22. The method according to any one of claims 10 to 21, wherein said non-human mammal is immunocompromised.

23. The method according to any one of claims 10 to 22, wherein said non-human mammal is a mouse.

24. The method according to claim 23 wherein said mouse is a SCID mouse.

25. The method according to any one of claims 10 to 21, wherein said non-human mammal is not immunocompromised.

26. A method for producing an autonomously growing cell displaying S-100, murine MHC class I, and secreting murine interleukin-6 comprising co-cultivating connective tissue fibroblasts obtained from a mammal with connective tissue diseases and fibroblasts from mice for at least 6 hours under sterile tissue culture conditions.

27. The method according to claim 26, wherein said connective tissue disease is an arthritis.

28. The method according to claim 26 or 27, wherein said cell has an aneuploid genotype.

29. The method according to any one of claim 26 to 28, wherein said one or more mammals are humans.

30. The method according to any one of claims 1 to 29, wherein the co-cultivation is effected for at least 12 weeks.

31. The method according to any one of claims 1 to 30, wherein said co-cultivation is effected in RPMI 1640 medium comprising 100 U/ml penicillin, 60 U/ml streptomycin and 10 % fetal calf serum.

32. The method according to any one of claims 1 to 31, wherein, at the outset of said co-cultivation, about 10⁶ murine and about 10⁶ human fibroblasts are seeded into a tissue culture flask.

33. The method according to any one of claims 1 to 32, wherein said cell is part of the cell line.

34. A cell produced by the method of any one of claims 1 to 33.

35. A kit comprising the cell according to any one of claim 1 to 8 or 34 or the cell line of claim 9 in one ore more containers.
